(19) Europäisches Patentamt
European Patent Office
Office européen des brevets

(11) EP 0 997 140 B1

(12) FASCICULE DE BREVET EUROPEEN

(45) Date de publication et mention de la délivrance du brevet:
**11.04.2001 Bulletin 2001/15**

(51) Int Cl.[7]: **A61K 7/48**

(21) Numéro de dépôt: **99402163.2**

(22) Date de dépôt: **30.08.1999**

# (54) Composition d'eclaircissement de la peau et/ou des cheveux

Haut- und/oder Haardepigmentierungsmittel

Skin and/or hair whitening composition

(84) Etats contractants désignés:
**AT BE CH CY DE DK ES FI FR GB GR IE IT LI LU MC NL PT SE**

(30) Priorité: **12.10.1998 FR 9812747**

(43) Date de publication de la demande:
**03.05.2000 Bulletin 2000/18**

(73) Titulaire: **L'OREAL**
**75008 Paris (FR)**

(72) Inventeur: **Touzan, Philippe**
**75012 Paris (FR)**

(74) Mandataire: **Renard, Emmanuelle**
**L'OREAL-DPI**
**6 rue Bertrand Sincholle**
**92585 Clichy Cedex (FR)**

(56) Documents cités:
**EP-A- 0 747 043** **US-A- 5 773 014**

- **WOODRUFF: "lightening skin and lessening cellulite" MANUFACTURING CHEMIST, avril 1996 (1996-04), pages 38-41, XP000683051**
- **PATENT ABSTRACTS OF JAPAN vol. 098, no. 001 (C & JP 09 227353 A (TSUMURA &AMP)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 997 140 B1

Printed by Jouve, 75001 PARIS (FR)

## Description

L'invention concerne une composition cosmétique et/ou dermatologique renfermant, en association, au moins un extrait de mûrier ou une substance active isolée à partir d'un tel extrait, au moins un extrait de scutellaire, ou une substance active isolée à partir d'un tel extrait, et au moins un dérivé d'acide salicylique. L'invention se rapporte également à l'utilisation de ladite composition par application topique sur la peau du visage et/ou du corps, dans le but de blanchir ou de dépigmenter la peau, de dépigmenter les poils et/ou les cheveux, ou de traiter les taches pigmentaires de la peau.

La couleur de la peau humaine est fonction de différents facteurs et notamment des saisons de l'année, de la race et du sexe, et elle est principalement déterminée par la nature et la concentration de mélanine produite par les mélanocytes. En outre, à différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kératinocytes situés à la surface de la peau.

De la même manière, la couleur des poils et des cheveux est due à la mélanine, lorsque les poils ou les cheveux sont foncés, certaines personnes désirent voir ceux-ci plus clairs. Ceci est particulièrement intéressant pour les poils qui sont moins visibles lorsqu'ils sont clairs que lorsqu'ils sont foncés.

Le mécanisme de formation de la pigmentation de la peau, des poils et des cheveux, c'est-à-dire de la formation de la mélanine, est particulièrement complexe et fait intervenir schématiquement les principales étapes suivantes :

$$\text{Tyrosine} \rightarrow \text{Dopa} \rightarrow \text{Dopaquinone} \rightarrow \text{Dopachrome} \rightarrow \text{Mélanine}$$

La tyrosinase (monophénol dihydroxyl phénylalanine : oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce à son activité hydroxylase et la réaction de transformation de la Dopa en dopaquinone grâce à son activité oxydase. Cette tyrosinase n'agit que lorsqu'elle est à l'état de maturation sous l'action de certains facteurs biologiques.

Une substance est reconnue comme dépigmentante si elle agit directement sur la vitalité des mélanocytes épidermiques où se déroule la mélanogénèse et/ou si elle interfère avec une des étapes de la biosynthèse de la mélanine soit en inhibant une des enzymes impliquées dans la mélanogénèse soit en s'intercalant comme analogue structural d'un des composés chimiques de la chaîne de synthèse de la mélanine, chaîne qui peut alors être bloquée et ainsi assurer la dépigmentation.

Les substances les plus utilisées en tant que dépigmentants sont plus particulièrement l'hydroquinone et ses dérivés, en particulier ses éthers tels que le monométhyléther et le monoéthyléther d'hydroquinone. Ces composés, bien qu'ils présentent une efficacité certaine, ne sont malheureusement pas exempts d'effets secondaires du fait de leur toxicité, ce qui peut rendre leur emploi délicat, voire dangereux. Cette toxicité provient de ce qu'ils interviennent sur des mécanismes fondamentaux de la mélanogénèse en tuant des cellules qui risquent alors de perturber leur environnement biologique et qui par conséquent obligent la peau à les évacuer en produisant des toxines.

Ainsi, l'hydroquinone est un composé particulièrement irritant et cytotoxique pour le mélanocyte, dont le remplacement, total ou partiel a été envisagé par de nombreux auteurs.

On a ainsi cherché des substances qui n'interviennent pas dans le mécanisme de la mélanogénèse mais qui agissent en amont sur la tyrosinase en empêchant son activation et sont de ce fait beaucoup moins toxiques.

L'utilisation de substances dépigmentantes topiques inoffensives présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne, telles que les taches pigmentaires séniles dites lentigo actiniques, les hyperpigmentations ou dépigmentations accidentelles, éventuellement dues à la photosensibilisation ou à la cicatrisation post-lésionnelle, ainsi que certaines leucodermies, telles que le vitiligo. Pour ces dernières (les cicatrisations pouvant aboutir à une cicatrice donnant à la peau un aspect plus blanc et les leucodermies), à défaut de pouvoir repigmenter la peau lésée, on achève de dépigmenter les zones de peau normale résiduelle pour donner à l'ensemble de la peau une teinte blanche homogène.

Aussi, il a été proposé divers agents blanchissants de la peau humaine, des poils et/ou des cheveux n'ayant pas les inconvénients des composés connus, c'est-à-dire qui sont non irritants, non toxiques et/ou non allergisants pour la peau et stables dans une composition.

Par exemple, le brevet EP-A-0 747 043 décrit l'utilisation de dérivés alkylés d'acide salicylique en tant qu'inhibiteurs de l'activité de la tyrosinase, dans une composition blanchissante ou dépigmentante. Le document US-A-5

580 549 décrit également l'utilisation de dérivés alkylés et alcoxylés de l'acide salicylique à fortes concentrations ou en association avec d'autres dépigmentants, pour traiter la dyschromie.

On connaît en outre de C.A. Essential Oils, Cosmetics, vol. 88, 1978, page 227, abrégé 88:65874z ou JP-A-50 135 236 des compositions cosmétiques dépigmentantes comprenant, à titre de principes actifs, des composés inhibant l'activité de la tyrosinase, issus d'extraits d'écorce des rejets ou des racines de mûrier, ou sohakuhi. Le document EP-A-0 296 923 divulgue également une composition à activité dépigmentante comprenant un extrait de mûrier, ou une substance active isolée à partir d'un tel extrait.

De son côté, le document JP06-107 532 concerne une composition cosmétique pour prévenir et traiter les taches pigmentaires en inhibant la mélanogénèse, laquelle composition renferme de 0,001 à 20% en poids d'un ou plusieurs extraits, dont un extrait de racine de Scutellaria baicalensis (scutellaire) et un extrait d'écorce de mûrier, et 0,001 à 30% en poids d'un absorbeur UV et/ou d'un agent diffusant les rayons UV.

Il subsiste toutefois le besoin d'une composition dépigmentante ou blanchissante qui, tout en étant bien tolérée, soit plus efficace que les compositions mentionnées ci-dessus.

Or, la Demanderesse a découvert que l'association d'extraits de mûrier et de scutellaire avec des dérivés d'acide salicylique potentialisait, de manière surprenante, l'efficacité de ces composés. En d'autres termes, ces trois composés, utilisés en combinaison, renforcent mutuellement leurs effets pour produire un effet de synergie dans le blanchiment ou la dépigmentation de la peau, des poils ou des cheveux.

Aussi, la présente invention a pour objet une composition cosmétique et/ou dermatologique renfermant, en association, au moins un extrait de mûrier ou une substance active isolée à partir d'un tel extrait, au moins un extrait de scutellaire, ou une substance active isolée à partir d'un tel extrait, et au moins un dérivé d'acide salicylique de formule (I) ou un sel d'un tel dérivé:

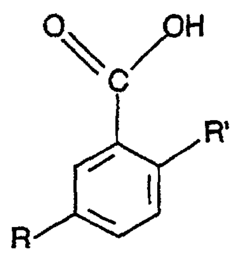

(I)

dans laquelle :

R représente un atome d'hydrogène ou une chaîne aliphatique, alcoxy, ester ou cétoxy, saturée ou insaturée, linéaire, ramifiée ou cyclisée, ces chaînes comportant de 2 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ; et

R' représente un groupement hydroxyle ou une fonction ester de formule :

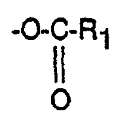

où $R_1$ est un groupement aliphatique saturé ou insaturé ayant de 1 à 18 atomes de carbone.

De préférence, le radical R comporte au moins 4 atomes de carbone. Il est par exemple formé d'un radical alkyle ou alcoxy linéaire saturé ayant de 4 à 11 atomes de carbone.

De façon avantageuse, le dérivé de l'acide salicylique est choisi parmi l'acide salicylique et les acides n-octanoyl-5-salicylique, n-décanoyl-5-salicylique et n-dodécanoyl-5-salicylique et il s'agit en particulier de l'acide n-octanoyl-5-salicylique.

Il peut s'agir également des sels de ces acides, et en particulier des sels obtenus par salification avec une base.

Comme bases susceptibles de salifier les dérivés de l'acide salicylique selon l'invention, on peut citer les

bases minérales comme les hydroxydes de métaux alcalins (hydroxydes de sodium et de potassium) ou les hydroxydes d'ammonium ou, mieux encore, les bases organiques.

De préférence, on utilise des bases amphotères pour la salification des dérivés de l'acide salicylique, c'est-à-dire des bases ayant à la fois des groupements fonctionnels anioniques et cationiques.

Les bases amphotères peuvent être des amines organiques primaires, secondaires, tertiaires ou cycliques, et plus spécialement des acides aminés. A titre d'exemple de bases amphotères, on peut citer la glycine, la lysine, l'arginine, la taurine, l'histidine, l'alanine, la valine, la cystéïne, la trihydroxyméthylaminométhane (TRISTA), la triéthanolamine. Ces bases sont utilisées en quantités suffisantes pour amener le pH de l'émulsion entre 5 et 7 et donc proche de celui de la peau. Il s'ensuit une grande compatibilité de la composition selon l'invention vis-à-vis de la peau.

Outre le dérivé d'acide salicylique précité, la composition selon l'invention renferme un extrait de mûrier, avantageusement un extrait de racine de Morus alba, ou une substance isolée à partir d'un tel extrait, de préférence une kuwanone ou un dérivé de celle-ci. Des procédés pour isoler la kuwanone E sont par exemple décrits dans Chemical Abstracts, vol. 89, 1978, abrégé 89:211925f et dans Heterocycles, vol. 9, n° 9, 1978.

La composition contient également un extrait de scutellaire, avantageusement un extrait de racine de Scutellaria baicalensis, ou une substance isolée à partir d'un tel extrait, de préférence de la baïcaline.

Les extraits mentionnés ci-dessus peuvent être préparés par extraction à l'aide d'un solvant aqueux, alcoolique ou organique, suivant toute méthode d'extraction connue de l'homme du métier

Par solvant aqueux on entend tout solvant constitué totalement ou pour part d'eau. On peut citer ainsi l'eau elle-même, les solvants hydroalcooliques en toute proportion ou encore les solvants constitués d'eau et d'un composé comme le propylène glycol ou le butylène glycol en toute proportion.

Parmi les solvants alcooliques on peut citer notamment l'éthanol.

On préfère, dans cette invention, utiliser des extraits de plantes obtenus par extraction à l'aide d'un solvant hydroalcoolique, avantageusement des extraits à 0,5-1,5% de matière active dans 50% d'eau et 48,5-49,5% de butylène glycol.

Quel que soit le mode de préparation utilisé selon l'invention, des étapes subséquentes visant à favoriser la conservation et/ou la stabilisation peuvent être ajoutées sans pour cela modifier la nature même de l'extrait. Ainsi, par exemple l'extrait obtenu peut être lyophilisé par toutes méthodes classiques de lyophilisation. On obtient ainsi une poudre qui peut être utilisée directement ou bien mélangée dans un solvant approprié avant utilisation.

La quantité de dérivé d'acide salicylique et d'extraits de mûrier et de scutellaire contenue dans la composition selon l'invention est bien évidemment fonction de l'effet de blanchiment ou de dépigmentation recherché et pourra de ce fait varier dans une large mesure. Dans une forme d'exécution préférée, la composition selon l'invention renferme de 0,1 à 5% en poids, avantageusement de 1 à 2% en poids, de dérivé d'acide salicylique ; et de 0,1 à 20% en poids, avantageusement de 0,5 à 1,5% en poids, de chacun des extraits de mûrier et de scutellaire, par rapport au poids total de la composition.

La composition selon l'invention est appropriée à une utilisation topique et contient donc un milieu cosmétiquement ou dermatologiquement acceptable, c'est-à-dire compatible avec la peau, les poils ou les cheveux.

La composition selon l'invention peut se présenter sous toutes les formes galéniques normalement utilisées pour une application topique, notamment sous forme d'une solution aqueuse, hydroalcoolique ou huileuse, d'une émulsion huile-dans-eau ou eau-dans-huile ou multiple, d'un gel aqueux ou huileux, d'un produit anhydre liquide, pâteux ou solide, d'une dispersion d'huile dans une phase aqueuse à l'aide de sphérules, ces sphérules pouvant être des nanoparticules polymériques telles que des nanosphères et des nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau ou sur les cheveux sous forme d'aérosol. Elle peut également se présenter sous forme solide, et par exemple sous forme de stick. Elle peut être utilisée comme produit de soin et/ou comme produit de maquillage. Elle peut également être sous une forme de shampooing ou d'après-shampooing.

De façon connue, la composition selon l'invention peut contenir également les adjuvants habituels dans les domaines cosmétique et dermatologique, tels que des gélifiants hydrophiles ou lipophiles, des actifs hydrophiles ou lipophiles, des conservateurs, des antioxydants, des solvants, des parfums, des charges, des filtres, des pigments, des absorbeurs d'odeur et des matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Bien entendu, il sera évident pour l'homme du métier que les composés, actifs ou non actifs, éventuellement ajoutés à la composition seront choisis de manière à ne pas contrevenir au but poursuivi par la présente invention.

Lorsque la composition selon l'invention est une émulsion, la proportion de la phase grasse peut aller de 5 à 80 % en poids, et de préférence de 5 à 50 % en poids par rapport au poids total de la composition. Les huiles, les

émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-20, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, on peut utiliser notamment les polyols, les vitamines, les agents kératolytiques et/ou desquamants, les agents anti-inflammatoires, les agents apaisants et leurs mélanges. On peut également ajouter à la composition selon l'invention d'autres agents dépigmentants, tels que l'acide kojique ou l'hydroquinone et ses dérivés, ce qui permet d'utiliser ces derniers à des doses moins élevées. En cas d'incompatibilité, certains au moins des actifs peuvent être incorporés dans des sphérules, notamment des vésicules ioniques ou non-ioniques et/ou des nanoparticules (nanocapsules et/ou nanosphères), de manière. à ce que les actifs incompatibles entre eux soient isolés les uns des autres dans la composition.

On peut aussi utiliser dans ces compositions des filtres UV à propriété lipophile ou hydrophile, tels que l'acide benzène 1,4-di(3-méthylidène-10-camphosulfonique), l'$\alpha$-cyano-$\beta$,-$\beta$-diphénylacrylate de 2-éthylhexyle ou octocrylène, le butyl méthoxydibenzoylméthane, l'octyl méthoxycinnamate et/ou les oxydes de titane et de zinc.

Ces compositions constituent notamment des crèmes de protection, de traitement ou de soin pour le visage, pour les mains ou pour le corps, des laits corporels de protection ou de soin, des lotions, gels ou mousses pour le soin ou le traitement de la peau, des lotions de nettoyage ou de désinfection, des compositions pour le bain, des fonds de teint et des crèmes teintées. Dans ces derniers cas, la composition contient des pigments.

La composition définie ci-dessus peut être utilisée pour dépigmenter et/ou blanchir la peau humaine et/ou enlever les taches pigmentaires de la peau et/ou dépigmenter les poils et/ou les cheveux ou pour inhiber l'activité de la tyrosinase et/ou la synthèse de la mélanine.

Aussi, l'invention se rapporte également à un procédé cosmétique de dépigmentation et/ou blanchiment de la peau humaine, des poils et/ou des cheveux, caractérisé en ce qu'il consiste à appliquer sur la peau, les poils et/ou les cheveux une composition selon l'invention.

L'invention va maintenant être illustrée à l'aide des exemples qui suivent. Les concentrations sont données en pourcentage en poids.

**Exemple 1 - Préparation d'une composition**

On a préparé la composition suivante :

| | | |
|---|---|---|
| **A -** | Glucate SS [Amerchol] (Methyl Glucose Sesquistearate) | 1,5% |
| | Alcools gras | 4% |
| | Cyclométhicone | 5% |
| | Acide n-octanoyl 5-salicylique 0,5% | |
| | Octocrylène (Uvinul N539) | 2% |
| | Conservateurs | 0,1% |
| | Parfum | 0,2% |
| **B -** | Eau qsp 100 | |
| | Glycérine | 3% |
| | Glucamate SSE-20 [Amerchol] (PEG-20 Methyl Glucose Sesquistearate) | 1,5% |

(suite)

| | | |
|---|---|---|
| | Conservateurs | 0,5% |
| C - | Eau | 15% |
| | Gomme de xanthanne | 0,1% |
| | Extrait hydroalcoolique de Racines de Mûrier (à 1 % de matière active) | 1% |
| | Extrait hydroalcoolique de scutellaire (à 1 % de matière active) | 1% |
| D - | Sepigel 305 (Seppic) | 1% |

La phase A est chauffée à 75°C jusqu'à parfaite solubilisation, ainsi que la phase B. La phase A est introduite dans la phase B sous agitation, jusqu'à obtention d'une émulsion fine et régulière. La phase C est homogénéisée sous agitation dans l'eau à 40°C puis introduite sous agitation à 40°C au mélange A + B. La phase D est introduite à 40°C et dispersée sous agitation.

Le fluide obtenu est refroidi sous agitation.

On obtient une émulsion fluide à propriété dépigmentante.

**Exemple 2 - Evaluation de l'effet dépigmentant de la composition de l'Exemple 1**

L'activité dépigmentante de la composition selon l'Exemple 1 a été mise en évidence sur un modèle de peau humaine maintenue en survie.

Pour ce faire, une peau humaine de phototype IV est obtenue en chirurgie plastique. Les fragments de peau sont maintenus en survie *ex vivo* par culture d'organe, c'est-à-dire qu'ils sont placés dans des inserts mis en suspension au-dessus de puits de culture et maintenus en survie à l'aide du milieu de culture déposé au fond des puits. Le milieu de culture est renouvelé trois fois par semaine.

Les produits ci-dessous sont appliqués une fois par jour sur les fragments ci-dessus, à raison de 2 mg/$cm^2$ de peau, cinq jours par semaine, pendant 21 jours :

Produit A l'excipient
Produit B l'excipient + 0,5 % d'acide n-octanoyl-5-salicylique
Produit C l'excipient + 1 % d'extraits végétaux (Mûrier + scutellaire)
Produit D l'excipient + 0,5 % d'acide n-octanoyl-5-salicylique + 1 % d'extraits végétaux (composition selon l'Exemple 1)

A titre de comparaison, un fragment de peau non traitée est également maintenu en survie dans les mêmes conditions.

Après 21 jours, une évaluation quantitative du pourcentage de cellules basales (kératinocytes et mélanocytes) renfermant des grains de mélanine est réalisée histologiquement au microscope optique, sur cinq champs, au grossissement 40, après coloration à l'hemalun-éosine (méthode Fontana).

Trois catégories de cellules sont dénombrées :

Score 1 : cellules faiblement pigmentées ou non pigmentées
Score 2 : cellules moyennement pigmentées, c'est-à-dire présentant une charge modérée en mélanine (grains de mélanine épars, dépôt non homogène)
Score 3 : cellules fortement pigmentées, c'est-à-dire présentant une charge importante en mélanine (dépôt homogène recouvrant plus de la moitié du cytoplasme des cellules).

Les résultats sont rassemblés dans le Tableau 1 suivant.

Tableau 1

| | Score 1 (%) | Score 2 (%) | Score 3 (%) |
|---|---|---|---|
| Peau non traitée | 5 | 34 | 67 |
| Peau traitée avec le produit A | 3 | 26 | 70 |
| Peau traitée avec le produit B | 4 | 30 | 66 |
| Peau traitée avec le produit C | 5 | 33 | 62 |
| Peau traitée avec le produit D | 10 | 51 | 39 |

Un indice de mélanine est calculé à partir de ce dénombrement suivant la formule IM =1 1 (Score 1) + 2 x (Score 2) + 3 x (Score 3).

A partir de cet indice, il est possible de calculer un pourcentage d'inhibition de mélanine :

$$\% \text{ d'inhibition} = \frac{IM\ (Te) - IM\ (Tr)}{IM\ (Te)} \times 100$$

où :

IM (Te) désigne l'Indice de Mélanine de la peau non traitée
IM (Tr) désigne l'Indice de Mélanine de la peau traitée avec le produit A, B, C ou D testé

Les résultats sont rassemblés dans le Tableau 2 suivant.

Tableau 2

| | Indice de Mélanine | % d'Inhibition |
|---|---|---|
| Peau non traitée | 274 | - |
| Peau traitée avec le produit A | 265 | 3 |
| Peau traitée avec le produit B | 262 | 4 |
| Peau traitée avec le produit C | 257 | 6 |
| Peau traitée avec le produit D | 229 | 16 |

Il n'y a pas de différence notable entre la peau non traitée et la peau traitée avec les produits A, B et C.
Par contre, par rapport à la peau non traitée ou à la peau traitée avec les produits A, B et C, la composition selon l'invention permet de diminuer significativement le nombre de cellules fortement pigmentées, d'augmenter le nombre de cellules moyennement pigmentées et d'obtenir ainsi un taux d'inhibition de la mélanine de 16%.

## Revendications

1. Composition cosmétique et/ou dermatologique renfermant, en association, au moins un extrait de mûrier ou une substance active isolée à partir d'un tel extrait, au moins un extrait de scutellaire, ou une substance active isolée à partir d'un tel extrait, et au moins un dérivé d'acide salicylique de formule (I) ou un sel d'un tel dérivé :

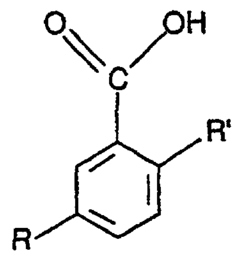

(I)

dans laquelle :

R représente un atome d'hydrogène ou une chaîne aliphatique alcoxy, ester ou cétoxy, saturée ou insaturée, linéaire, ramifiée ou cyclisée, ces chaînes comportant de 2 à 22 atomes de carbone et pouvant être substituées par au moins un substituant choisi parmi les atomes d'halogène, le groupement trifluorométhyle, les groupements hydroxyle sous forme libre ou estérifiée par un acide ayant de 1 à 6 atomes de carbone ou bien par une fonction carboxyle, libre ou estérifiée par un alcool inférieur ayant de 1 à 6 atomes de carbone ; et

R' représente un groupement hydroxyle ou une fonction ester de formule :

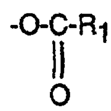

où $R_1$ est un groupement aliphatique saturé ou insaturé ayant de 1 à 18 atomes de carbone.

2. Composition selon la revendication 1, caractérisée en ce que le dérivé d'acide salicylique est choisi parmi les acides salicylique, n-octanoyl-5-salicylique, n-décanoyl-5-salicylique et n-dodécanoyl-5-salicylique.

3. Composition selon la revendication 1 ou 2, caractérisée en ce que le dérivé d'acide salicylique est l'acide n-octanoyl-5 salicylique.

4. Composition selon l'une quelconque des revendications précédentes, caractérisée en ce que ledit dérivé d'acide salicylique ou son sel est présent en une concentration allant de 0,1 à 5 % en poids, et de préférence en une concentration de 1 à 2% en poids, par rapport au poids total de la composition.

5. Composition selon l'une quelconque des revendications précédentes, caractérisée en ce qu'au moins l'un desdits extraits est un extrait hydroalcoolique.

6. Composition selon l'une quelconque des revendications précédentes, caractérisée en ce que ledit extrait de mûrier est un extrait de racine de Morus alba.

7. Composition selon l'une quelconque des revendications précédentes, caractérisée en ce que ledit extrait de scutellaire est un extrait de racine de Scutellaria baïcalensis.

**8.** Composition selon l'une quelconque des revendications précédentes, caractérisée en ce qu'elle renferme de 0,1 à 20% en poids de chacun desdits extraits, et de préférence de 0,5 à 1,5% en poids de chacun desdits extraits, par rapport au poids total de la composition.

**9.** Composition selon l'une quelconque des revendications 1 à 6, caractérisée en ce que ladite substance active isolée à partir de l'extrait de scutellaire est la baïcaline.

**10.** Composition selon l'une quelconque des revendications 1 à 7 et 9, caractérisée en ce que ladite substance isolée à partir de l'extrait de mûrier est une kuwanone ou un dérivé de celle-ci.

**11.** Composition selon l'une quelconque des revendications précédentes, caractérisée en ce qu'elle contient un milieu cosmétiquement et/ou dermatologiquement acceptable.

**12.** Utilisation cosmétique d'une composition selon l'une quelconque des revendications précédentes pour dépigmenter et/ou blanchir la peau humaine et/ou enlever les taches pigmentaires de la peau et/ou dépigmenter les poils et/ou les cheveux.

**13.** Utilisation d'une composition selon l'une quelconque des revendications précédentes pour la fabrication d'une préparation destinée à dépigmenter et/ou blanchir la peau humaine et/ou enlever les taches pigmentaires de la peau et/ou dépigmenter les poils et/ou les cheveux.

**14.** Utilisation d'une composition selon l'une quelconque des revendications 1 à 11 pour la fabrication d'une préparation destinée à inhiber l'activité de la tyrosinase et/ou la synthèse de la mélanine.

**15.** Procédé cosmétique de dépigmentation et/ou blanchiment de la peau humaine, des poils et/ou des cheveux, caractérisé en ce qu'il consiste à appliquer sur la peau, les poils et/ou les cheveux une composition selon l'une quelconque des revendications 1 à 11.

**Patentansprüche**

**1.** Kosmetische und/oder dermatologische Zusammensetzung, die in Kombination mit mindestens einem Brombeerextrakt oder einem aus diesem Extrakt abgetrennten Wirkstoff, mindestens einem Helmkrautextrakt oder einem aus diesem Extrakt abgetrennten Wirkstoff und mindestens ein Salicylderivat der Formel (I) oder ein Salz dieses Derivats enthält:

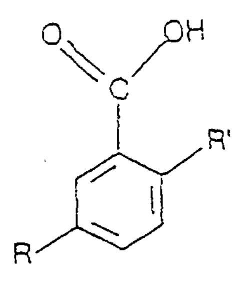

(I)

worin bedeuten:

R Wasserstoff oder eine aliphatische gesättigte oder ungesättigte, geradkettige, verzweigte oder cyclische Alkoxy-, Ester- oder Cetoxygruppe; wobei die Gruppen 2 bis 22 Kohlenstoffatome aufweisen und mit mindestens einem Substituenten substituiert sein können, der unter den Halogenatomen, Trifluormethyl, Hydroxygruppen, die in freier Form vorliegen oder mit einer $C_{1-6}$-Säure verestert sind, oder einer Carboxygruppe, die in freier Form vorliegen und einer Carbonylgruppe, die in freier Form vorliegt oder mit einem niederen $C_{1-6}$-Alkohol verestert ist, ausgewählt ist; und
R' eine Hydroxygruppe oder eine Estergruppe der Formel:

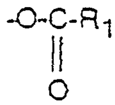

worin $R_1$ eine gesättigte oder ungesättigte aliphatische Gruppe mit 1 bis 18 Kohlenstoffatomen bedeutet.

2. Zusammensetzung nach Anspruch 1, dadurch gekennzeichnet, daß das Salicylsäurederivat unter 5-N-Octanoylsalicylsäure, 5-N-Decanoylsalicylsäure und 5-N-Dodecanoylsalicylsäure ausgewählt ist.

3. Zusammensetzung nach Anspruch 1 oder 2, dadurch gekennzeichnet, daß das Salicylsäurederivat die 5-N-Octanoylsalicylsäure ist.

4. Zusammensetzung nach einem der vorhergehenden Ansprüche, dadurch gekennzeichnet, daß das Salicylsäurederivat oder sein Salz in einer Konzentration von 0,1 bis 5 Gew.-% und vorzugsweise 1 bis 2 Gew.-%, bezogen auf das Gesamtgewicht der Zusammensetzung, vorliegt.

5. Zusammensetzung nach einem der vorhergehenden Ansprüche, dadurch gekennzeichnet, daß mindestens ein Extrakt ein wäßrig-alkoholischer Extrakt ist.

6. Zusammensetzung nach einem der vorhergehenden Ansprüche, dadurch gekennzeichnet, daß der Brombeerextrakt ein Extrakt der Wurzel von Morus alba ist.

7. Zusammensetzung nach einem der vorhergehenden Ansprüche, dadurch gekennzeichnet, daß der Helmkrautextrakt ein Extrakt der Wurzel von Scutellaria baicalensis ist.

8. Zusammensetzung nach einem der vorhergehenden Ansprüche, dadurch gekennzeichnet, daß sie 0,1 bis 20 Gew.-% jedes Extrakt und vorzugsweise 0,5 bis 1,5 % jedes Extrakts, bezogen auf das Gesamtgewicht der Zusammensetzung, enthält.

9. Zusammensetzung nach einem der Ansprüche 1 bis 6, dadurch gekennzeichnet, daß der aus dem Helmkrautextrakt abgetrennte Wirkstoff das Baicalin ist.

10. Zusammensetzung nach einem der Ansprüche 7 und 9, dadurch gekennzeichnet, daß der aus dem Brombeerextrakt abgetrennte Wirkstoff das Kuwanon oder ein Kuwanonderivat ist.

11. Zusammensetzung nach einem der vorhergehenden Ansprüche, dadurch gekennzeichnet, daß sie ein kosmetisch und/oder dermatologisches Medium enthält.

12. Kosmetische Verwendung einer Zusammensetzung nach einem der vorhergehenden Ansprüche zur Depigmentierung und/oder zum Bleiben der menschlichen Haut und/oder zum Entfernen von Pigmentflecken der Haut und/oder um Körperhaare und/oder Haare zu depigmentieren.

13. Verwendung einer Zusammensetzung nach einem der vorhergehenden Ansprüche zur Herstellung eines Präparats, das zur Depigmentierung und/oder zum Bleichen der menschlichen Haut und/oder zum Entfernen von Pigmentflecken der Haut und/oder zur Depigmentierung von Körperhaaren und/oder Haaren vorgesehen ist.

14. Verwendung einer Zusammensetzung nach einem der Ansprüche 1 bis 11 zur Herstellung eines Präparats, das zur Inhibierung der Tyrosinaseaktivität und/oder der Melaninsynthese vorgesehen ist.

15. Kosmetisches Verfahren zur Depigmentierung und/oder zum Bleichen der menschlichen Haut, der Körperhaare und/oder der Haare, das dadurch gekennzeichnet ist, daß es darin besteht, auf die haut, die Körperhaare und/oder die Haare eine Zusammensetzung nach einem der Ansprüche 1 bis 11 aufzutragen.

**Claims**

**1.** Cosmetic and/or dermatological composition containing, in combination, at least one extract of mulberry or an active substance isolated from such an extract, at least one extract of skullcap or an active substance isolated from such an extract, and at least one salicylic acid derivative of formula (I) or a salt of such a derivative:

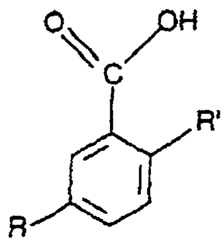

(I)

in which:

R represents a hydrogen atom or a saturated or unsaturated, linear, branched or cyclic aliphatic, alkoxy, ester or ketoxy chain, these chains comprising from 2 to 22 carbon atoms and possibly being substituted with at least one substituent chosen from halogen atoms, a trifluoromethyl group and hydroxyl groups in free form or esterified with an acid containing from 1 to 6 carbon atoms or else with a carboxyl function which is free or esterified with a lower alcohol containing from 1 to 6 carbon atoms; and
R' represents a hydroxyl group or an ester function of formula:

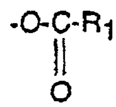

in which $R_1$ is a saturated or unsaturated aliphatic group containing from 1 to 18 carbon atoms.

**2.** Composition according to Claim 1, characterized in that the salicylic acid derivative is chosen from salicylic acid, 5-n-octanoylsalicylic acid, 5-n-decanoylsalicylic acid and 5-n-dodecanoylsalicylic acid.

**3.** Composition according to Claim 1 or 2, characterized in that the salicylic acid derivative is 5-n-octanoylsalicylic acid.

**4.** Composition according to any one of the preceding claims, characterized in that the said salicylic acid derivative or its salt is present in a concentration ranging from 0.1 to 5% by weight, and preferably in a concentration of 1 to 2% by weight, relative to the total weight of the composition.

**5.** Composition according to any one of the preceding claims, characterized in that at least one of the said extracts is an aqueous-alcoholic extract.

**6.** Composition according to any one of the preceding claims, characterized in that the said extract of mulberry is an extract of the roots of Morus alba.

**7.** Composition according to any one of the preceding claims, characterized in that the said extract of skullcap is an extract of the roots of Scutellaria baicalensis.

**8.** Composition according to any one of the preceding claims, characterized in that it contains from 0.1 to 20% by weight of each of the said extracts, and preferably from 0.5 to 1.5% by weight of each of the said extracts, relative to the total weight of the composition.

**9.** Composition according to any one of Claims 1 to 6, characterized in that the said active substance isolated from the extract of skullcap is baicaline.

**10.** Composition according to any one of Claims 1 to 7 and 9, characterized in that the said substance isolated from the extract of mulberry is kuwanone or a derivative thereof.

**11.** Composition according to any one of the preceding claims, characterized in that it contains a cosmetically and/or dermatologically acceptable medium.

**12.** Cosmetic use of a composition according to any one of the preceding claims for depigmenting and/or whitening human skin and/or for removing skin pigmentation marks and/or for depigmenting head hair and/or other hairs.

**13.** Use of a composition according to any one of the preceding claims for the manufacture of a preparation intended for depigmenting and/or whitening human skin and/or for removing skin pigmentation marks and/or for depigmenting head hair and/or other hairs.

**14.** Use of a composition according to any one of Claims 1 to 11 for the manufacture of a preparation intended to inhibit tyrosinase activity and/or melanin synthesis.

**15.** Cosmetic process for depigmenting and/or whitening human skin, head hair and/or other hairs, characterized in that it consists in applying a composition according to any one of Claims 1 to 11 to the skin, head hair and/or other hairs.